# EUROPÄISCHE PATENTSCHRIFT

(11) **EP 3 634 205 B1**
(45) Veröffentlichungstag und Bekanntmachung des Hinweises auf die Patenterteilung: **13.01.2021**
(21) Anmeldenummer: 18728582.0
(22) Anmeldetag: 28.05.2018
(51) Int. Cl.: A61B 5/00, H04R 25/00

(54) **VORRICHTUNG ZUR SENSORISCHEN ERFASSUNG WENIGSTENS EINES MENSCHLICHEN VITALPARAMETERS**
DEVICE FOR DETECTING AT LEAST ONE HUMAN VITAL PARAMETER BY MEANS OF A SENSOR
DISPOSITIF POUR ENREGISTRER À L'AIDE D'UN CAPTEUR AU MOINS UN PARAMÈTRE VITAL HUMAIN

(30) Priorität: 09.06.2017 DE 102017209767
(43) Veröffentlichungstag der Anmeldung: 15.04.2020
(73) Patentinhaber: Neuroloop GmbH, 79108 Freiburg (DE)
(72) Erfinder: KIMMIG, Fabian, 79108 Freiburg (DE); BORETIUS, Tim, 79108 Freiburg (DE); PLACHTA, Dennis, 79279 V rstetten (DE)
(74) Vertreter: Rösler, Uwe
(86) Internationale Anmeldenummer: PCT/EP2018/063863
(87) Internationale Veröffentlichungsnummer: WO 2018/224340

(56) Entgegenhaltungen:
- WO-A2-2016/069866

## Beschreibung

### Technisches Gebiet

Die Erfindung bezieht sich auf eine Vorrichtung zur sensorischen Erfassung wenigstens eines menschlichen Vitalparameters mit einem Träger, der geeignet geformt und dimensioniert ist, um zumindest teilweise in den menschlichen äußeren Gehörgang herausnehmbar platziert zu werden, und an dem wenigstens ein Sensor zur Erfassung eines Vitalparameters angebracht ist.

### Stand der Technik

Gattungsgemäße Vorrichtungen sind leicht- und kleinbauende Einheiten, die zur sensoriellen Vitalparametererfassung zur Anbringung am Ohr unter der Maßgabe ausgebildet sind, dass sie einerseits für die Person keine Einschränkungen oder Behinderungen darstellen und andererseits kaum oder nicht wahrnehmbar für Dritte in Erscheinung treten. Bekannte, am bzw. im Ohr anzubringende Sensorsysteme vermögen Vitalparameter, wie bspw. Blutdruck, Blutsauerstoffsättigung, EKG-Signal, Herzfrequenz u. ä. sensoriell zu erfassen und zu Zwecken einer weiteren Datenauswertung kabellos oder kabelgebunden an eine Datenaufzeichnungs- sowie auch Auswerteeinheit zu übertragen.

Aus der Druckschrift EP 2 116 138 B1 ist ein robustes optoelektronisches, im Ohr positionierbares kardiovaskuläres Überwachungsgerät zu entnehmen, das zu Zwecken einer sicheren Anbringung am Ohr einen hinter der Ohrmuschel anzuordnenden, ergonomisch angepassten Hakenabschnitt aufweist, der mit einem zumindest teilweise in den äußeren Gehörgang einmündenden Gehäuseteil verbunden ist und somit den Gehörgang mechanisch sowie auch akustisch verschließt. Neben einem im Hakenabschnitt integrierten Bewegungssensor ist wenigstens ein optischer Emitter vorgesehen, dessen emittiertes Licht auf die rückseitige Ohrmuschel abstrahlt. Die durch die Ohrmuschel transmittierenden Lichtanteile werden von einem am Gehäuseteil angebrachten optischen Empfänger detektiert und im Weiteren einer photoplethysmographischen Auswertung unterzogen, die in einer externen Auswerteeinheit erfolgt, an die die detektierten Lichtsignale drahtgebunden oder drahtlos übermittelt werden. Optional können am Hakenabschnitt und/oder am Gehäuseteil zusätzliche Signal- und Empfangselektroden für die Erfassung elektrokardiologischer Signale vorgesehen sein.

Gleichwohl das bekannte am Ohr anzubringende Sensorsystem den Anforderungen hinsichtlich Tragekomfort, Bewegungsfreiheit und optische Unauffälligkeit weitgehend gerecht wird, schließt der pfropfenartig, in den äußeren Gehörgang einmündende Gehäuseteil den Gehörgang schall- und luftdicht ab, wodurch die akustische Wahrnehmung des betreffenden Ohres zumindest stark beeinträchtigt ist.

Die Druckschrift US 6,454,718 B1 offenbart einen zylinderförmigen Sensorträger, der zum Einstecken in den äußeren Gehörgang geeignet ausgebildet ist und über eine Vielzahl von Sensoren verfügt, so bspw. ein Dehnungssensor zur Blutdruckmessung, Sauerstoffsensor, etc..

Die Druckschrift EP 1 594 340 A2 beschreibt einen trichterförmigen Halter zum Einsetzen in den menschlichen äußeren Gehörgang, in den ein akustischer Übertragungskanal zum Anschluss an ein Hörgerät anschließbar ist.

Gleichfalls besteht in den beiden vorstehend beschriebenen Fällen kein freier Zugang in den äußeren Gehörgang, vielmehr wird dieser durch die jeweils beiden bekannten Vorrichtungen akustisch dicht abgeschlossen.

In der Druckschrift US 2003/0233051 A1, in der eine Variante des vorstehenden Überwachungsgerätes beschrieben ist, wird der Umstand der akustischen Abkopplung durch den in den äußeren Gehörgang hineinragenden Gehäuseteil dazu genutzt bzw. dadurch vermieden, indem ein Lautsprecher in den Gehäuseteil integriert ist, der bspw. mit einer akustischen Signalquelle, bspw. in Form eines MP3-Players oder eines Mikrofons zu Zwecken der Übertragung von Umgebungsgeräuschen verbunden ist.

Die Druckschrift WO 2016/069866 A2 beschreibt einen interaktiven Gehörstöpsel, der eine hohle konische Form besitzt, an dessen distalen Konusspitze ein Lautsprecher angebracht ist.

### Darstellung der Erfindung

Der Erfindung liegt die Aufgabe zugrunde eine Vorrichtung zur sensorischen Erfassung wenigstens eines menschlichen Vitalparameters mit einem Träger, der geeignet geformt und dimensioniert ist, um zumindest teilweise in den menschlichen, äußeren Gehörgang herausnehmbar platziert zu werden und an dem wenigstens ein Sensor zur Erfassung eines Vitalparameters angebracht ist, derart weiterzubilden, dass der Tragekomfort weiter verbessert sein soll. Insbesondere gilt es den äußeren Gehörgang akustisch nicht zu verschließen, so dass auf mögliche die Umgebungsgeräusche übertragende bzw. verstärkende Systeme verzichtet werden kann. In gleicher Weise gilt es die optische Unauffälligkeit zu bewahren bzw. zu verbessern.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Lösungsgemäß ist eine Vorrichtung zur sensorischen Erfassung wenigstens eines menschlichen Vitalparameters nach den Merkmalen des Oberbegriffes des Anspruches 1 dadurch ausgebildet, dass der Träger, auf oder in dem wenigstens ein Sensor zu Erfassung eines Vitalparameters angebracht ist, in Form eines Flächensubstrates ausgebildet ist, das in Art eines Hohlzylinders geformt ist, der einen durchgängig offenen Hohlkanal radial zu einer dem Hohlzylinder zugeordneten Zylinderachse nach außen begrenzt und einen Hohlzylinderaußenwandbereich aufweist, der im Zustand der Platzierung des Trägers zumindest teilweise in den menschlichen äußeren Gehörgang in Flächenkontakt mit der Gehörganginnenwand tritt. Das Flächensubstrat ist durch Wickeln um eine Wickelachse in die Gestalt des Hohlzylinders überführbar und verfügt über eine materialinhärente formerhaltende Steifigkeit, so dass das durch Wickeln in Art eines Hohlzylinders umgeformte Flächensubstrat zwei einander gegenüberliegende Flächensubstratabschnitte verfügt, die sich wicklungsbedingt und ohne äußeren mechanischen Zwang gegenseitig lose überlappen.

Durch die Ausbildung des Trägers in Art eines dünnwandigen Hohlzylinders, der sich im eingefügten Zustand eng an die Gehörganginnenwand anschmiegt, bleibt der freie Zugang des äußeren Gehörganges im Wesentlichen unbeeinflusst. Auf diese Weise wird für die Dauer des in den äußeren Gehörgang eingesetzten hohlzylinderförmigen Trägers die akustische Wahrnehmung der betreffenden Person nicht beeinträchtigt. Auch bleibt der natürliche Stimulus zum Druckausgleich bei abrupten Druckänderungen unverändert erhalten.

In einer bevorzugten Ausführungsform besteht der Träger aus einem hautfreundlichen Flächensubstrat, vorzugsweise aus einem ein- oder mehrlagigen, folienartig ausgebildeten Polymermaterial, das durch Wickeln um eine Wickelachse in die Gestalt eines Hohlzylinders überführbar ist. Das folienartige Flächensubstrat verfügt typischerweise über eine Substratdicke von 10 µm bis einige wenige 100 µm und schließt im gewickeltem Zustand einen Hohlzylinder ein, dessen Hohlzylinderinnendurchmesser vernachlässigbar kleiner ist als der Durchmesser des menschlichen äußeren Gehörganges, der typischerweise zwischen 4 und 7 mm beträgt. Selbst bei einer mehrfachen Umwicklung des Flächensubstrat um die Wickelachse und einer damit verbundenen, sich mehrlagig ausbildenden Folienüberlappung kann eine sich dabei ausbildende Hohlzylinderwanddicke in Hinblick auf die Dimensionen des menschlichen Gehörganges vernachlässigt werden, so dass mit dem Einsatz der lösungsgemäßen Vorrichtung in den äußeren Gehörgang keine oder nur vernachlässigbar geringe akustische Einschränkungen verbunden sind und darüber hinaus für eine weitgehend unveränderte Belüftung des äußeren Gehörganges gesorgt ist.

Vorzugsweise ist das Flächensubstrat im nicht gewickelten Zustand quadratisch oder rechteckförmig und verfügt über eine Ober- und Unterseite. Auch sind weitere Flächensubstratformen denkbar, wie sie in Zusammenhang mit den Figuren erläutert werden. In Abhängigkeit des jeweiligen messtechnischen Anforderungsprofils sind in oder auf das folienartige Flächensubstrat eine bestimmte Anzahl sowie unterschiedliche Arten von Sensoren zu integrierten bzw. zu applizieren, deren Aufgabe es ist, physiologische Vitalparameter von einer Person zu erfassen. Vorzugsweise bietet sich hierzu die an sich bekannte Dünnschichttechnik an, mit der es möglich ist mikrosystemtechnische Sensoren in bzw. auf das Flächensubstrat zu integrieren bzw. zu applizieren. Die Flexibilität und das darauf beruhende Wickelvermögen des vorzugsweise aus einem mehrlagigen, folienartigen Polymermaterial gefertigten Flächensubstrates wird durch die Sensoren nicht oder nur unwesentlich beeinträchtigt, so dass eine nachfolgende Wicklung des mit Vitalsensoren besetzten bzw. konfektionierten Flächensubstrates zu einem Hohlzylinder möglich ist.

Der Wickelvorgang erfolgt vorzugsweise parallel zu einer Seitenkante des quadratisch oder rechteckförmigen Flächensubstrates derart, so dass das durch Wickeln umgeformte Flächensubstrat in Art eines Hohlzylinder zwei einander gegenüberliegende Flächensubstratabschnitte verfügt, die sich wicklungsbedingt und ohne äußeren mechanischen Zwang gegenseitig lose überlappen. Das Maß der gegenseitigen Überlappung ist grundsätzlich beliebig wählbar. Wie bereits erwähnt, ist es aus Gründen einer möglichst geringfügigen Beeinträchtigung des äußeren Gehörganges vorteilhaft, das Flächensubstrat unter Ausbildung einer einlagigen Hohlzylinderwand im Wege einer Wicklung umzuformen, so dass sich die gegenüberliegenden Flächensubstratabschnitte im Wege der Wicklung lediglich geringfügig überlappen.

Gleichsam ist es möglich, das Flächensubstrat durch mehrmaliges, spiralförmiges Umwickeln um eine Wickelachse zur Ausbildung einer mehrlagigen Hohlzylinderwand umzuformen.

Um zu vermeiden, dass sich das vorzugsweise aus Polyimid bestehende, folienartige Flächensubstrat, das als Träger für die Sensorik dient, nach dem Wickeln selbstständig in den Ausgangszustand zurückformt, wird das zu einem Hohlzylinder gewickelte Flächensubstrat einer Wärmebehandlung unterzogen, wodurch das Flächensubstrat eine materialinhärente formerhaltende Steifigkeit erhält und seine Hohlzylinderform langzeitstabil beibehält.

In allen genannten möglichen Ausführungsformen, umfasst das zu einem Hohlzylinder geformte Flächensubstrat einen hohlzylindrischen Durchgangskanal, durch den der äußere Gehörgang belüftet bleibt und somit akustisch vollständig an die Umgebung ankoppelt. Darüber hinaus bleiben auf diese Weise der natürliche Druckausgleich sowie auch ein erzwungener Druckausgleich möglich.

Zu Zwecken der Vitalparametererfassung bietet sich eine Reihe unterschiedlicher Sensoren zur Integration in bzw. Applikation auf das Flächensubstrat an. So lässt sich bspw. die Herzfrequenz mit Hilfe eines kapazitiv wirkenden Sensorelementes, bspw. in Ausbildung einer innerhalb des Flächensubstrates integrierten Interdigitalelektrodenstruktur realisieren. Gilt es die Körpertemperatur zu erfassen, so bieten sich hierzu auf elektrische Widerstandsänderung basierende Temperatursensoren an, bspw. PT 100. Zur Erfassung der Blutsauerstoffsättigung eignen sich geeignet gewählte Leuchtmittel und entsprechend gewählte Photodetektoren, die zu Zwecken der Lichtemission sowie auch Detektion an einer dem äußeren Gehörgang zugewandten Oberseite des Trägers bzw. Flächensubstrates angeordnet sind und Messsignale zur Auswertung auf der Grundlage der Photoplethysmographie zu generieren vermögen.

Ferner bieten sich an der dem äußeren Gehörgang zugewandten Oberfläche des Flächensubstrates angebrachte Elektrodenkontakte an, um elektrokardiographische Signale über den Hautkontakt zur Gehörinnenwand abzugreifen, um ein Elektrokardiogramms (EKG) erstellen zu können. Überdies können im Flächensubstrat weitere Sensoren, wie bspw. Beschleunigungssensoren etc. integriert sein.

In einer weiteren Ausführungsvariante ist am Träger wenigstens ein Aktor angebracht, vorzugsweise in Form eines Schall erzeugenden Aktors. So lassen sich manche Formen von Tinnitus durch akustisches Überlagern mit einem künstlich erzeugten Rauschen eines bestimmten Rauschspektrums in den psychoakustischen Hintergrund zurückdrängen. Ein derartiges Rauschen kann mit Hilfe eines "Miniaturlautsprechers", der in das Flächensubstrat integriert ist, vorzugsweise mit einer zur Hohlzylinderinnenwand zugewandten Schallabstrahlungsfläche realisieren. Ein solcher im Flächensubstrat integrierter Aktor hätte den Scharm, dass die AußenGeräusche ungehindert zum Ohr gelangen können und dennoch kontinuierlich gegen den Tinnitus "gearbeitet" werden kann. Derartige Aktoren bzw. Schalltransducer stellen vorzugsweise kleine Keramikelemente z.B. PZT, dar.

Sämtliche in oder auf dem Träger integrierte bzw. applizierte Sensoren sowie gegebenenfalls Aktoren sind vorzugsweise kabelgebunden mit dem Elektronikmodul verbunden, in dem neben einer elektrischen Energiequelle eine für die Signalaufbereitung und -auswertung erforderliche Auswerteelektronik enthalten ist. Das Elektronikmodul lässt sich vorzugsweise als separate Baueinheit zum Träger unauffällig hinter der Ohrmuschel mit Hilfe einer ergonomisch an das Ohr angepasste Halterung fixieren.

Alternativ oder in Kombination bietet es sich an in den hohlzylinderförmigen Träger eine zur Energie- und Signalübertragung geeignete Empfangs- und Sendeeinheit zu integrieren, bspw. auf Basis der RFID-Technik, so dass es möglich ist, sämtliche im bzw. auf dem Träger platzierten Sensoren drahtlost zu betreiben. Hierzu bedarf es einer zusätzlichen, im Träger integrierten Mikroelektronikeinheit, an die elektrische Energie sowie Steuersignale von einem externen Steuergerät zur Versorgung und Ansteuerung der Sensoren übertragen werden, und über zudem die die sensorisch erfassten Vitalparameter in Form von Sensorsignalen an das Steuergerät zur weiteren Auswertung übertragen werden. Durch Nutzung einer derartigen drahtlosen Signalübertragungstechnik bedarf es lediglich eines zuverlässig innerhalb des äußeren Gehörganges fest sitzenden, hohlzylinderartig ausgebildeten Sensorträgers, der ansonsten nach außen hin für Dritte visuell nicht wahrnehmbar ist.

In einer besonders bevorzugten Ausführungsform kann die Funktion des externen Steuergerätes ein von der Person mitgeführtes handelsübliches Smartphone übernehmen, das hardwareseitig ohnehin über sämtliche Komponenten verfügt, die für eine drahtlose Energie- und Signalübertragung an die Träger-seitig integrierte Mikroelektronikeinheit nötig sind. Zur technischen Befähigung des Smartphones mit dem im externen Gehörgang platzierten, hohlzylinderartigen Sensorträger zu Zwecken der Vitalparametererfassung und -übermittlung zu kommunizieren, kann durch Installation eines geeignet hierfür konzipierten Programmes, in Form einer App, vorgenommen werden.

Die lösungsgemäße Vorrichtung eignet sich insbesondere für die Erfassung der nachfolgenden menschlichen Vitalparameter: Herzfrequenz, EKG-Signal, Körpertemperatur, Blutsauerstoffsättigung, CO₂-Blut-Sättigung, Blutzucker, pH-Wert, Hautwiderstand sowie Blutdruck.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a: schematisierte Darstellung eines ebenen Flächensubstrates mit Vitalparametersensoren;
- Fig. 1b: Darstellungen des gewickelten Flächensubstrates in Art eines Hohlzylinders,
- Fig. 2: schematisierte Darstellung des hohlzylinderartig ausgebildeten Sensorträgers platziert im äußeren Gehörgang,
- Fig. 3: einstückige Ausführung des zylinderartigen Sensorträgers als Hohlzylinder (nicht Gegenstand der Erfindung) sowie
- Fig. 4a, b, c: Sensorträger mit strukturiertem Flächensubstrat.

### Wege zur Ausführung der Erfindung, gewerbliche Verwendbarkeit

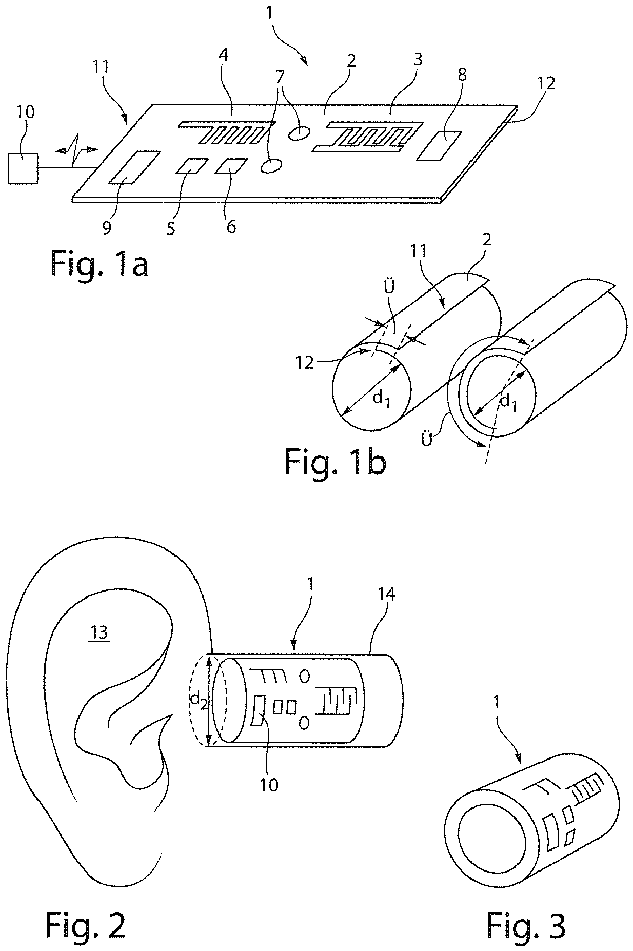

Figur 1a zeigt eine perspektivische Draufsicht eines schematisch dargestellten Trägers 1 in Form eines eben ausgebildeten, rechteckförmigen Flächensubstrats 2 in Form einer ein- oder mehrlagigen Polymerschicht, die typischerweise eine Schichtdicke von wenigen 10 µm bis wenigen 100 µm besitzt. Auf dem Flächensubstrat 2 bzw. in diesem integriert/appliziert ist eine Anzahl unterschiedlicher Vitalparametersensoren. Die nachfolgenden Vitalparametersensoren 3,...,8 können in beliebiger Anzahl und Anordnung auf bzw. im Flächensubstrat 2 angebracht sein. Auch ist es möglich lediglich einen einzigen Vitalparametersensor auf oder in dem Flächensubstrat 2 vorzusehen. Geeignete Vitalparametersensoren sind: Kapazitive Interdigitalstruktur 3 zur Erfassung der Herzfrequenz, Widerstandsbasierter Temperatursensor 4, vorzugsweise PT100 oder PT1000 Temperaturfühler, zur Erfassung der Körpertemperatur; LED-Photodiode 5 sowie Photodetektor 6 zur Messung der Blutsauerstoffsättigung bzw. CO₂-Blutsättigung auf Basis der Photoplethysmographie, EKG-Sensoren 7 in Form von an der Oberfläche des Flächensubstrates angebrachter Kontaktelektroden, Beschleunigungssensor 8 sowie entsprechend weitere mikroelektronische Sensoren, die allesamt vorzugsweise in Dünnschichttechnologie auf das Flächensubstrat 2 appliziert bzw. in dieses integriert sind. Sämtliche auf dem Flächensubstrat 2 vorhandenen Vitalparametersensoren sind mit einer Mikroelektronikeinheit 9 über nicht dargestellte elektrische Leiterbahnen elektrisch verbunden, über die die Vitalparametersensoren sowohl mit Energie als auch Steuersignale versorgt werden und über die die erfassten Sensorsignale zur weiteren Verarbeitung der Mikroelektronikeinheit 9 zugeleitet werden. Die Mikroelektronikeinheit 9 ist in einem Ausführungsbeispiel kabelgebunden mit einem externen Steuergerät 10 verbunden, das sowohl zur Energie- als auch Signalversorgung dient.

In einer besonders bevorzugten Ausführungsform erfolgt die Signal- und Energieübertragung zwischen dem Steuergerät 10 und der Mikroelektronik 9 auf Basis der RFID-Technik oder einer ähnlichen drahtlosen Energie und Signalübertragungstechnik, so dass das Steuergerät 10 als mobile Einheit getrennt vom Träger 1 gehandhabt werden kann. Alternativ bietet sich auch eine kabelgebundene Lösung an.

Figur 1b zeigt das Flächensubstrat 2 in einer zu einem Hohlzylinder gewickelten Form, in der die sich gegenüberliegenden Seitenkantenbereiche 11, 12 des Flächensubstrats 2 geringfügig überlappen, siehe Überlapp Ü, linke Darstellung in Figur 1b. Alternativ ist es möglich, die Wicklung des Flächensubstrats 2 mit einer großflächigen gegenseitigen Überlappung Ü der sich gegenüberliegenden Seitenkantenbereiche 11, 12 gemäß der rechten Darstellung in Figur 1b auszubilden.

Nach der Wicklung des aus einem Polymer bestehenden folienartigen Flächensubstrats 2, gemäß den in Figur 1b dargestellten Hohlzylinderformen, erfolgt eine Behandlung des gewickelten Flächensubstrats 2 derart, so dass das Flächensubstrat 2 eine materialinhärente formerhaltende Steifigkeit erfährt, durch die die Hohlzylinderform langzeitstabil erhalten bleibt, bspw. im Wege eines Tempervorganges.

Das zu einem Hohlzylinder gewickelte Flächensubstrat 2 weist einen Zylinderdurchmesser d1 auf, der etwas größer bemessen ist als der Innendurchmesser d2 des äußeren Gehörganges G eines Menschen, siehe hierzu auch Figur 2, die schematisiert eine menschliche Ohrmuschel 13 mit einem äußeren Gehörgang 14, in den ein Sensorträger 1 eingefügt ist, zeigt. Durch den kleineren Innendurchmesser d2 wird der hohlzylinderförmige Sensorträger 1 im Sitz innerhalb des Gehörganges 14 radial etwas komprimiert, wodurch sich eine radial auf die Gehörinnenwand des äußeren Gehörganges 14 wirkende Anpresskraft ausbildet, die den Sensorträger 1 lösbar fest und sicher im Gehörgang 14 fixiert.

Ferner befinden sich all jene Vitalparametersensoren, zu deren Funktion Hautkontakt zur Gehörganginnenwand oder zumindest ein direkter optischer Zugang erforderlich ist an der radial nach außen orientierten Oberseite des hohlzylinderförmig geformten Trägers 1, diese betreffen insbesondere die EKG-Kontaktflächen 7 sowie die Leuchtdiode 5 sowie der Photodetektor 6 des Sauerstoffsättigungssensors 5, 6. Hingegen sind der Körpertemperatursensor 4 sowie der Herzfrequenzsensor 3 von außen unsichtbar innerhalb des Flächensubstrats 2 integriert. Nur aus Gründen der Illustration der einzelnen Sensoren sind die Sensoren 3 bis 8 allesamt sichtbar auf der Oberfläche des Flächensubstrats 2 in Figur 1a dargestellt.

Figur 2 zeigt den Zustand des hohlzylinderförmigen Sensorträgers 1 innerhalb des externen Gehörganges 14 eines Menschen, wobei sich die radial äußere Zylinderoberfläche druckbeaufschlagt an die Innenwand des äußeren Gehörganges 14 flächenkontaktierend anschmiegt. Das mit der Mikroelektronik 9 bspw. über eine Drahtverbindung verbundene Steuergerät 10 kann miniaturisiert ausgeführt und unscheinbar hinter der Ohrmuschel 13 befestigt werden. Alternativ ist es möglich, das Steuergerät 10 bei Nutzung einer drahtlosen Kommunikationstechnik zur Mikroelektronik 9 als externe Einheit, bspw. in Form eines Smartphones auszubilden.

Die in Figur 3 dargestellte Ausführungsform eines hohlzylinderförmig ausgebildeten Sensorträgers 1 stellt einen einstückig ausgebildeten Hohlzylinder dar, der nicht Gegenstand der Erfindung ist und aus einem kompressiblen Material, bspw. einem hautfreundlichen Polymerschaum besteht. Gleichsam dem Einfügen eines an sich bekannten Ohrstöpsels kann auf diese Weise auch der mit Sensoren bestückte Träger 1 radial komprimiert und in den äußeren Gehörgang 14 einer Person eingeschoben werden. der hohlzylinderförmige Träger 1 gemäß Figur 3 ist im Wege eines Gießverfahrens hergestellt.

Bei allen Ausführungsformen für eine Vorrichtung zur sensorischen Erfassung menschlicher Vitalparameter innerhalb des äußeren Gehörganges 14 bleibt der Gehörgang 14 aufgrund der hohlzylinderförmigen Ausbildung des Trägers 1 durchgängig belüftet, sodass auch ein dauerhafter Einsatz der Vorrichtung im Ohr weder zu hygienischen Problemen noch zu sonstigen Gehöreinschränkungen führt.

Der sich an die Innenwand des äußeren Gehörganges druckbeaufschlagt radial anschmiegende, hohlzylinderförmige Sensorträger 1 besitzt als gerader Hohlzylinder über seine gesamte axial Hohlzylindererstreckung einen konstanten Außendurchmesser. Da die natürliche Innenkontur des äußeren Gehörganges nicht notwendigerweise der Mantelfläche eines Geradzylinderförmig entspricht, kann es vorkommen, dass der hohlzylinderförmige Sensorträger keinen gleichmäßigen Flächenkontakt zum äußeren Gehörgang besitzt. Dies kann dazu führen, dass Pulswellen-bedingte Verformungen des äußeren Gehörganges, die mittels der vorstehend beschriebenen kapazitiven Interdigitalstruktur gemessen werden können, durch nur teilweisen Kontakt zwischen dem Sensorträger und der Innenwand des äußeren Gehörganges nicht vollständig erfasst werden können.

Um dies zu vermeiden bietet es sich das Flächensubstrat geeignet zu formen bzw. zu strukturieren, um eine durch Wicklung des Flächensubstrats individuelle Hohlform zu erhalten, die sich möglichst passgenau an die Innenwand des äußeren Gehörganges anzuschmiegen vermag.

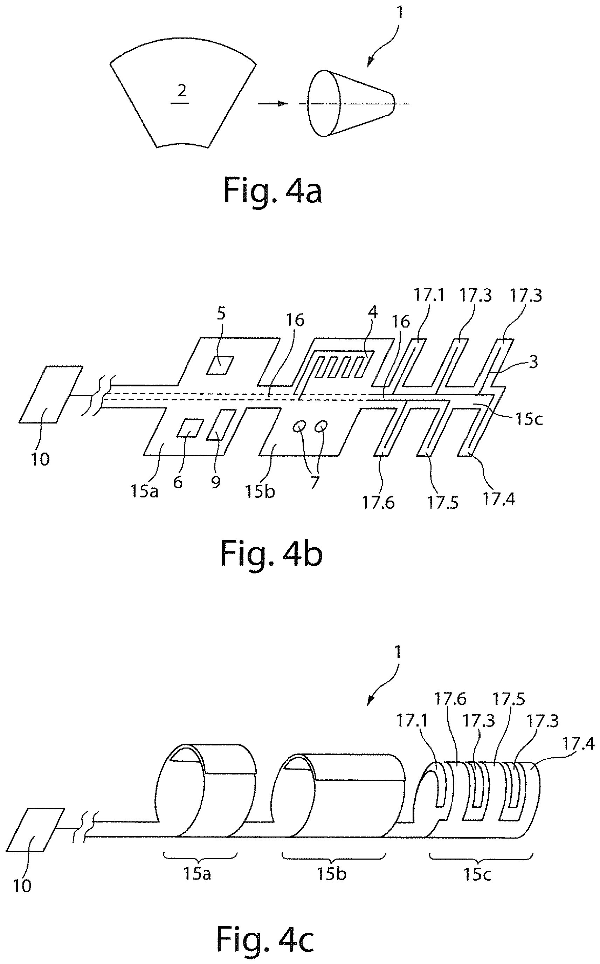

Fig. 4 a zeigt hierzu ein Flächensubstrat 2 in planarer, linke Darstellung, und in gewickelter Form, rechte Darstellung. Das planare Flächensubstrat 2 besitzt die Form eines Ringteilstückes, das in gewickelter Form einen Trichter bildet zum Einsetzen in den äußeren Gehörgang. Auf die Darstellungen der im Sensorträger 1 enthaltenen Sensoren ist verzichtet.

Figur 4b zeigt ein weiteres Ausführungsbeispiel mit einem strukturierten Flächensubstrat 2, das über Zwischenstegbereiche 16 miteinander einstückig verbundene Flächensegmente 15a, 15b , 15c besitzt. Die Flächensegmente 15a, 15b, 15c können in Form und Größe voneinander abweichen. Im Unterschied zu den jeweils rechteckförmig ausgebildeten planaren Flächensegmenten 15a und 15b ist das Flächensegment 15 c zusätzlich strukturiert ausgeführt, um im gewickelten Zustand individuell radial abspreizbare Fingerabschnitte 17.1, 17.2, 17.3, 17.4, 17.5 etc. zu besitzen, längs derer jeweils zur kapazitiven Herzfrequenzmessung Interdigitalelektroden 3 eingebracht sind. Durch die im Vergleich zu einer Hohlzylinderwand unterbrochene Fingerstruktur im Flächensegment 15c wird eine individuelle Anlage der einzelnen Fingerabschnitte an die individuelle Geometrie der Innenwand des äußeren Gehörganges möglich.

Der Wickelvorgang zur Überführung des planaren Flächensubstrats in die gewickelte Form erfolgt im Rahmen eines Temperprozesses, um die hohlzylinderförmige Gestalt dauerhaft zu bewahren.

Je nach individuellen Gegebenheiten können die Zwischenstegbereiche 16 in Breite und Länge geeignet gewählt werden. Auch die Anbringung und Verteilung der Sensoren, wie EKG-Kontaktflächen 7, Leuchtdiode 5 sowie Photodetektor 6 des Sauerstoffsättigungssensors 5, 6, Körpertemperatursensor 4 sowie die Mikrosteuerelektronik 9, können bedarfsweise vorgenommen werden.

Der Sensorträger 1 kann auch in diesem Fall drahtgebunden oder drahtlos mit einem Steuergerät 10 verbunden sein, wie bereits vorstehend erwähnt.

### Bezugszeichenliste

- 1: Träger
- 2: Flächensubstrat
- 3: Herzfrequenzsensor
- 4: Körpertemperatursensor
- 5, 6: Sauerstoffsättigungssensor bzw. CO₂-Sensor
- 7: EKG-Sensor
- 8: weitere Sensoren
- 9: Mikroelektronikeinheit
- 10: Steuergerät
- 11: Seitenkantenbereich
- 12: Seitenkantenbereich
- 13: Ohrmuschel
- 14: äußerer Gehörgang
- 15a: Flächensegment
- 15b: Flächensegment
- 15c: Flächensegment
- 16: Zwischenstegbereich
- 17.1...: Fingerabschnitte
- 17.6: Fingerabschnitte
- d1: Hohlzylinderdurchmesser im Zustand ohne äußeren Zwang
- d2: Durchmesser des äußeren Gehörgangs

## Patentansprüche

1. Vorrichtung zur sensorischen Erfassung wenigstens eines menschlichen Vitalparameters mit einem Träger (1), der geeignet geformt und dimensioniert ist, um zumindest teilweise in den menschlichen äußeren Gehörgang (14) herausnehmbar platziert zu werden, und an dem wenigstens ein Sensor (3-8) zur Erfassung eines Vitalparameters angebracht ist,
wobei der Träger (1) in Art eines Hohlzylinders geformt ist, einen durchgängig offenen Hohlkanal radial zu einer dem Hohlzylinder zugeordneten Zylinderachse nach außen begrenzt und einen Hohlzylinderaußenwandbereich aufweist, der ausgebildet ist um im Zustand der Platzierung des Trägers (1) zumindest teilweise in dem menschlichen äußeren Gehörgang (14) in Flächenkontakt mit diesem zu treten,
**dadurch gekennzeichnet, dass** der Träger ein Flächensubstrat (2) ist, das durch Wickeln um eine Wickelachse in die Gestalt des Hohlzylinders überführbar ist, und dass das Flächensubstrat (2) über eine materialinhärente formerhaltende Steifigkeit verfügt, so dass das durch Wickeln in Art eines Hohlzylinders umgeformte Flächensubstrat (2) zwei einander gegenüberliegende Flächensubstratabschnitte verfügt, die sich wicklungsbedingt und ohne äußeren mechanischen Zwang gegenseitig lose überlappen.

2. Vorrichtung nach Anspruch 1,
**dadurch gekennzeichnet, dass** das in Art eines Hohlzylinders gewickelte Flächensubstrat (2) ohne äußeren mechanischen Zwang einen ersten äußeren Zylinderdurchmesser (d1) aufweist, der größer bemessen ist als ein zweiter äußerer Zylinderdurchmesser (d2), den das in Art eines Hohlzylinders gewickelte Flächensubstrat im Zustand der Platzierung des Trägers zumindest teilweise in dem menschlichen äußeren Gehörgang besitzt, wobei das Flächensubstrat eine radial nach außen gerichtete Kraft auf den äußeren Gehörgang ausübt.

3. Vorrichtung nach Anspruch 1 oder 2,
**dadurch gekennzeichnet, dass** das Flächensubstrat (2) wenigstens zwei jeweils über einen Zwischenstegbereich (16) einstückig miteinander verbundenen Flächensegmente (15a, 15b) aufweist, und
dass die Flächensegmente (15a, 15b) jeweils in Art eines Hohlzylinders gewickelt sind.

4. Vorrichtung nach Anspruch 3,
**dadurch gekennzeichnet, dass** wenigstens ein Flächensegment (15c) strukturiert ist und im gewickelten Zustand individuell radial abspreizbare und jeweils paarweise ineinandergreifende Fingerabschnitte (17.1, 17.2, 17.3, 17.4, 17.5, 17.6) aufweist.

5. Vorrichtung nach einem der Ansprüche 1 bis 4,
**dadurch gekennzeichnet, dass** das Flächensubstrat (2) aus einem ein- oder mehrlagigen Polymermaterial gefertigt ist, in dem der wenigstens eine Sensor (3-8) integriert oder auf dem der wenigstens eine Sensor (3-8) appliziert ist.

6. Vorrichtung nach einem der Ansprüche 1 bis 5,
**dadurch gekennzeichnet, dass** der wenigstens eine Sensor (3-8) aus der nachfolgenden Gruppe ausgewählt ist: Interdigital-Elektrodenanordnung, elektrischer Widerstands-Sensor, aus Lichtquelle und Lichtdetektor bestehendes Lichtsensor-System, Beschleunigungssensor.

7. Vorrichtung nach einem der Ansprüche 1 bis 6,
**dadurch gekennzeichnet, dass** an einem dem äußeren Gehörgang (14) zugewandten Hohlzylinderaußenwandbereich wenigstens ein freiliegender, metallischer Oberflächenkontakt (7) vorgesehen ist, der mit einer innerhalb des Flächensubstrats (2) integrierten elektrischen Komponente (9) verbunden ist.

8. Vorrichtung nach einem der Ansprüche 1 bis 7,
**dadurch gekennzeichnet, dass** am Träger (1) eine Signal- und Energieübertragungseinheit (9) angebracht ist, die kabellos oder kabelgebunden mit einem externen Endgerät (10) in Signal- und Energieaustausch steht.

9. Vorrichtung nach einem der Ansprüche 1 bis 8,
**dadurch gekennzeichnet, dass** der Träger (1) derart ausgebildet und dimensioniert ist, dass im Zustand der Platzierung des Trägers (1) zumindest teilweise in dem menschlichen externen Gehörgang (14) ein freier Zugang zum Trommelfell durch den externen Gehörgang (14) besteht.

10. Vorrichtung nach Anspruch 4 und 6,
**dadurch gekennzeichnet, dass** die Interdigital-Elektrodenanordnung in dem strukturierten Flächensegment (15c) integriert ist.

11. Vorrichtung nach einem der Ansprüche 1 bis 10,
**dadurch gekennzeichnet, dass** an dem Träger (1) wenigstens ein Aktor angebracht ist.

12. Verwendung der Vorrichtung nach einem der Ansprüche 1 bis 11 zur Erfassung wenigstens einen der nachfolgenden menschlichen Vitalparameter:
Herzfrequenz, EKG-Signal, Körpertemperatur, Blutsauerstoffsättigung, CO2-Sensor, pH-Wert, Blutzucker, Hautwiderstand, Blutdruck.

## Claims

1. A device for the recording by sensors of at least one human vital parameter, with a carrier (1), which is suitably shaped and dimensioned to be placed in a removable manner at least partially in the human external auditory canal (14) and on which at least one sensor (3-8) for recording a vital parameter is applied wherein the carrier (1) is in the form of a hollow cylinder, outwardly delimits a continuously open hollow channel radially to a cylinder axis assigned to the hollow cylinder, and comprises a hollow cylinder outer wall area which in the state of placement of the carrier (1) at least partially in the human auditory canal (14) is configured to come into contact therewith,
**characterised in that** the carrier is a surface substrate (2) which through winding around a winding axis can be transformed in the form of the hollow cylinder, and **in that** the surface substrate (2) has a material-inherent shape-retaining stiffness so that the surface substrate (2) transformed by winding into the form of a hollow cylinder has two surface substrate regions lying opposite each other which due to winding and without external mechanical force, loosely overlap each other.

2. The device according to claim 1,
**characterised in that** without external mechanical force, the surface substrate (2) wound into the form of a hollow cylinder has a first outer cylinder diameter (d1) which is dimensioned to be greater than a second outer cylinder diameter (d2) which the surface substrate wound into the form of a hollow cylinder has when the carrier is at least partially placed in the human external auditory canal, wherein the surface substrate exerts a radially outwardly directed force onto the external auditory canal.

3. The device according to claim 1 or 2,
**characterised in that** the surface substrate (2) comprises at least two surface segments (15a, 15b) connected to each other in one piece by means of an intermediate web area (16) and
**in that** the surface segments (15a, 15b) are each wound into the form of a hollow cylinder.

4. The device according to claim 3,
**characterised in that** at least one surface segment (15c) is structured and in the wound state comprises individually radially spreadable finger sections (17.1, 17.2, 17.3, 17.4, 17.5, 17.6) each interlocking in pairs.

5. The device according to any one of claims 1 to 4,
**characterised in that** the surface substrate (2) is made of a single or multiple-layer polymer material, into which the at least one sensor (3-8) is integrated or onto which the at least one sensor (3-8) is applied.

6. The device according to any one of claims 1 to 5,
**characterised in that** the at least one sensor (3-8) is selected from the following group: interdigital electrode device, electrical resistance sensor, light sensor system consisting of a light source and light detector, acceleration sensor.

7. The device according to any one of claims 1 to 6, **characterised in that** provided on a hollow cylinder outer wall area facing the external auditory canal (14) is at least one exposed, metallic surface contact (7) which is connected to an electrical component (9) integrated into the surface substrate (2).

8. The device according to any one of claims 1 to 7,
**characterised in that** on the carrier (1) a signal and energy transmission unit (9) is applied, which exchanges signals and energy with an external terminal device (10) in a wireless or wire-connected manner.

9. The device according to any one of claims 1 to 8,
**characterised in that** the carrier (1) is configured and dimensioned in such a way that when the carrier (1) is placed at least partially in the human auditory canal (14) there is free access to the eardrum through the external auditory canal (14).

10. The device according to claim 4 and 6,
**characterised in that** the interdigital electrode arrangement is integrated into the structured surface segment (15c).

11. The device according to any one of claims 1 to 10,
**characterised in that** at least one actuator is applied to the carrier (1).

12. Use of the device according to any one of claims 1 to 11 for recording at least one of the following human vital parameters: heart frequency, ECG signal, body temperature, blood oxygen saturation, CO₂ sensor, pH value, blood sugar, skin resistance, blood pressure.

## Revendications

1. Dispositif de saisie par capteur d'au moins un paramètre vital humain avec un support (1), qui est formé et dimensionné de façon appropriée pour être placé au moins en partie, pouvant être enlevé, dans le conduit auditif externe humain (14) et sur lequel est disposé au moins un capteur (3-8) pour la saisie d'un paramètre vital,
sachant que le support (1) est formé à la manière d'un cylindre creux, limite vers l'extérieur un conduit creux généralement ouvert radialement par rapport à un axe de cylindre attribué à un cylindre creux et comporte une zone de paroi extérieure de cylindre creux qui est constituée pour entrer dans l'état de positionnement du support (1) au moins en partie dans le conduit auditif externe humain (14) en contact superficiel avec celui-ci,
**caractérisé en ce que** le support est un substrat superficiel (2), qui peut être transformé par enroulement autour d'un axe d'enroulement dans la forme du cylindre creux et **en ce que** le substrat superficiel (2) dispose d'une rigidité maintenant la forme inhérente au matériau de telle manière que le substrat superficiel (2) façonné par enroulement à la manière d'un cylindre creux fournit deux sections de substrat superficiel opposées l'une à l'autre qui se chevauchent librement mutuellement, conditionnées par l'enroulement et sans contrainte mécanique extérieure.

2. Dispositif selon la revendication 1,
**caractérisé en ce que** le substrat superficiel (2) enroulé à la manière d'un cylindre creux sans contrainte mécanique extérieure comporte un premier diamètre de cylindre extérieur (d1) qui est dimensionné plus grand qu'un deuxième diamètre de cylindre (d2) extérieur, qui possède le substrat superficiel enroulé à la manière d'un cylindre creux dans l'état de positionnement du support au moins en partie dans le conduit auditif externe humain, sachant que le substrat superficiel exerce une force radialement orientée vers l'extérieur sur le conduit auditif externe.

3. Dispositif selon la revendication 1 ou 2,
**caractérisé en ce que** le substrat superficiel (2) comporte au moins deux segments superficiels (15a, 15b) reliés respectivement l'un à l'autre en une pièce par une zone à nervure intermédiaire (16) et
**en ce que** les segments superficiels (15a, 15b) sont enroulés respectivement à la manière d'un cylindre creux.

4. Dispositif selon la revendication 3,
**caractérisé en ce qu'**au moins un segment superficiel (15c) est structuré et comporte à l'état enroulé des sections digitales (17.1, 17.2, 17.3, 17.4, 17.5, 17.6) individuellement écartables radialement et s'emboîtant l'une dans l'autre par paire.

5. Dispositif selon l'une quelconque des revendications 1 à 4,
**caractérisé en ce que** le substrat superficiel (2) est fabriqué à partir d'un matériau polymère à couche unique ou multiple dans lequel est intégré au moins un capteur (3-8) ou sur lequel est appliqué au moins un capteur (3-8).

6. Dispositif selon l'une quelconque des revendications 1 à 5,
**caractérisé en ce qu'**au moins un capteur (3-8) est choisi à partir du groupe suivant : montage d'électrodes interdigital, capteur à résistance électrique, système de capteur de lumière composé d'une source lumineuse et d'un détecteur de lumière, capteur d'accélération.

7. Dispositif selon l'une quelconque des revendications 1 à 6,
**caractérisé en ce que** sur une zone de paroi extérieure de cylindre creux tournée vers le conduit auditif externe (14) est prévu au moins un contact de surface (7) métallique libre, qui est relié à un composant (9) électrique intégré à l'intérieur du substrat superficiel (2).

8. Dispositif selon l'une quelconque des revendications 1 à 7,
**caractérisé en ce que** sur le support (1) est disposée une unité de transmission de signaux et d'énergie (9), qui est en échange de signaux et d'énergie sans câble ou relié par câble avec un terminal externe (10).

9. Dispositif selon l'une quelconque des revendications 1 à 8,
**caractérisé en ce que** le support (1) est constitué et dimensionné de telle manière que dans l'état de positionnement du support (1), il existe au moins en partie dans le conduit auditif externe (14) un accès libre à la membrane du tympan par le conduit auditif externe (14).

10. Dispositif selon les revendications 4 et 6,
**caractérisé en ce que** le montage d'électrodes interdigital est intégré dans le segment superficiel structuré (15c).

11. Dispositif selon l'une quelconque des revendications 1 à 10,
**caractérisée en ce qu'**au moins un actionneur est disposé sur le support (1).

12. Utilisation du dispositif selon l'une quelconque des revendications 1 à 11 pour la saisie d'au moins un des paramètres vitaux humains suivants ; fréquence cardiaque, signal d'ECG, température corporelle, saturation en oxygène du sang, capteur de CO2, valeur du pH, glycémie, résistance cutanée, pression sanguine.
